# EUROPEAN PATENT SPECIFICATION

(11) **EP 3 079 566 B1**
(45) Date of publication and mention of the grant of the patent: **29.04.2020**
(21) Application number: 14869071.2
(22) Date of filing: 11.12.2014
(51) Int. Cl.: A61B 5/00, A61B 5/06, A61M 35/00

(54) **LIGHT THERAPY PLATFORM SYSTEM**
LICHTTHERAPIE-PLATTFORMSYSTEM
SYSTÈME DE PLATE-FORME DE LUMINOTHÉRAPIE

(30) Priority: 11.12.2013 US 201361914624 P; 07.07.2014 US 201414324453
(43) Date of publication of application: 19.10.2016
(73) Proprietor: JOHNSON & JOHNSON CONSUMER INC., Skillman, NJ 08558 (US)
(72) Inventor: TAPPER, Jay, Wayne, PA 19087 (US); BLAUSTEIN, Lawrence A., Chagrin Falls, OH 44022 (US); SHUTER, David, Palm Beach Gardens, FL 33418 (US); FREITAG, Eric, Brooklyn, NY 11201 (US); ALTHOFF, Charles Peter, New York, NY 10036 (US); BRAMLEY, Alistair, Brooklyn, NY 11211 (US); ZADEH, Allen, Brooklyn, NY 11201 (US); SHUTER, Daniel Joseph, Palm Beach Gardens, FL 33418 (US); LORENC, Zbigniew Paul, New York, NY 10028 (US)
(74) Representative: Carpmaels & Ransford LLP
(86) International application number: PCT/US2014/069789
(87) International publication number: WO 2015/089302

(56) References cited:
- US-A1- 2004 044 384
- US-A1- 2004 162 549
- US-A1- 2005 070 977
- US-A1- 2006 217 787
- US-A1- 2009 143 842
- US-A1- 2013 066 404
- US-A1- 2013 066 404
- US-B1- 6 290 713
- US-B1- 6 290 713
- US-B2- 7 520 630

## Description

### FIELD

The present embodiments relate to devices for delivering light-based skin therapy treatments for improving skin health, such as anti-aging enhancement or acne prevention, using light-emitting diode (LED) light therapy, although other types of light radiating sources can be used.

### BACKGROUND

Certain light spectrums emitted by LEDs (blue or red) are known to be therapeutic for skin treatment against maladies such as acne, or are beneficial to inhibit skin aging. However, there is a need to provide users/patients with a convenient at-home light therapy delivery device such as a wearable mask, veil or hood that is adjustable or flexible to conform to different sizes and shapes, and that is simple to use without user discomfort. Currently available at-home, consumer usable products on the market are fixed to one-size and/or usually have to be hand-held; which generally have not proven satisfactory for providing the best or desired light dispersion. The alternative is customers visiting a doctor's office to receive treatments.

Prior known light therapy devices, particularly masks, have suffered from problems relating to the exposure of the LEDs and the associated circuitry to power the LEDs to contact by users. More particularly, in an effort to maximize light communication to a patient, the LEDs have been disposed in a manner which allow them to be physically engaged (e.g., touched) by a patient, or even contact a treatment surface, which processes are debilitating to the LEDs as a result of the accumulation of dirt and oil. In addition, any such engagement can be dangerous to patients who are exposed to the sharp or hot edges of the LEDs and the associated circuitry. The exposure of detailed circuitry presents an intimidating and unpleasant experience when the therapy requires several minutes of time for completion and the mask is disposed relatively close to the face, often causing an uncomfortable, claustrophobic sensation over time to the patient.

A hands-free therapeutic experience is always better than having to hold the device in a particular position for extended periods of time during the therapy. Numerous assemblies have been conceived for mounting masks and helmet-like devices to varieties of straps, bands, wraps and cords, which can result in a pressing of the support and mounting assembly closely against the hair or scalp of a patient. There is always a need to minimize the extent of such attachment assemblies so that on the one hand the subject device is securely attached on the patient, but also that the attaching structure has minimal consequence to the patient's comfort during the therapy itself. Being relatively light in weight, and easily and minimally supported during therapeutic use are important to consumer acceptance.

As users come in a variety of shapes and sizes, devices should be size or area adjustable so that the therapy can be efficiently applied and/or selectively intensified to desired treatment areas.

Lastly, particularly in therapeutic devices treating facial areas, eye protection is needed to avoid light damage or irritation to a patient's eyes. Prior known devices have typically used separable patches which must rest on the eye area to block the therapeutic light from communication to the eye system itself. There is a need for a better way that is readily adaptable to communicate therapeutic light to areas near the eyes, particularly with regard to anti-aging treatments, and still protect the patient.

It is desired to provide alternative means of using the benefits of the light therapy in a manner to maximize therapeutic efficiencies in exposure while maintaining ease and convenience of use. For this reason, a variety of light weight, flexible and adjustable embodiments are disclosed within this disclosure incorporating a variety of energy varying applications responsive to user conditions or needs.
US2013/0066404A1 describes a light therapy platform system comprising a first wall, to which the lamps are affixed thereto and a second wall, closer to the patient, spaced from the first wall wherein the lamps are recessed relative thereto. The second wall comprises a reflective surface facing towards a patient and a plurality of light apertures substantially aligned with the LEDs on the first wall for communicating lamp radiation from the lamps to the user.
US2005/0070977A1 describes a light and magnetic emitting mask including light emitting diodes and electromagnets embedded in the mask.
US2004/0162549A1 describes a method and apparatus for performing optical dermatology having a mount adapter for positioning in proximity of a patient's skin, one or more radiation sources disposed in the mount for irradiating at least a portion of the area of the patient's skin, and a control circuitry electrically coupled to the radiation sources for actuating a selected pattern or sequence of the radiation sources for performing a treatment protocol.
US2006/0217787A1 describes a light therapy device including apertures to allow the passage of air flow. The inside edges of the apertures could be coated with a reflective layer.

### SUMMARY

The invention is as defined in the appended claims. The present embodiments comprise phototherapy systems and devices comprising a therapeutic lamp platform for radiant lamps such as LEDs are disposed in an assembly comprising a first wall to which the lamps are affixed thereto and a second wall, closer to the patient, spaced from the first wall wherein the lamps are recessed relative thereto. The second wall comprises a reflective surface facing towards a patient and a plurality of light apertures substantially aligned with the LEDs on the first wall for communicating lamp radiation from the lamps to a user. The lamps and associated circuitry are disposed between the first and second wall so that the reflective surface is relatively smooth and seamless towards the patient. The number of lamps are minimized, as is the circuitry therefor, and other assembly materials are purposefully selected for a relatively light weight assembly resulting in enhanced user comfort during therapy sessions. The walls have a malleable rigidity for flexible adjustability relative to the user. More particularly, the walls have a concave configuration relative to the face of the user which is adjustable relative to a rest position to be expandable relative to a size of the head of the user for a close fitting and secure engagement to the user during use. The device is mounted to the user with a frame comprising an eyeglass frame or goggles including lenses for shielding the user's eyes from lamp radiation. The adjustability of the embodiments is further enhanced by the walls being pivotable relative to the support frame and where the frames may include telescopic temple arms for selective adjustability relative to the head size of the user. The device is thus supported on the patient as a wearable hands-free mask or the like. A power source communicates energy to the lamps and comprises a remote battery pack and may also include a control processor for counting the number of uses by the device for the user and for indicating a need for device replacement after a predetermined number of uses.

The present embodiments comprise an adjustable/flexible platform for providing a light-based therapy that is adaptable to the user's receptive surfaces, whether based on size or condition, wherein the light therapy can be applied without limitation of the kind of light and without limitation of the ultimate purpose of the therapy, i.e., beauty, health, and/or wound healing. Such sources can vary in the form of the radiant energy delivery. Pulsed light (IPL), focused light (lasers) and other methods of manipulating light energy are encompassed within the present embodiments. Other methods of light emission may comprise continuous, pulsed, focused, diffuse, multi wavelength, single wavelength, visible and/or non-visible light wavelengths.

A present embodiment describes forms such as a shaped/fitted mask, goggles, eye mask, shroud or hood, and facial mask (collectively referred to as "mask") with LED light emitted from LED bulbs or LED strips that are capable of being adjusted to accommodate the variances in face size or areas intended for therapeutic attention. Control systems are included to vary light intensity, frequency or direction.

The platform can be secured to the head by multiple means: eyeglass frames, straps, drawstring, harness, velcro, turn dial or snap and buttons. As the mask is secured it can be adjusted upward, for chin to forehead coverage. It can also be adjusted outward, for side-to-side coverage. In addition, once the platform has been bent/slid to cover the face area, the distance of the platform from the skin can be adjusted for achieving a desired light intensity relative to a user's skin surface. Thus, the light therapy can be maximized in up to three physical dimensions.

The subject adjustability may be implemented through "smart" processing and sensor systems for enhanced flexibility/adjustability in the form of adjustable energy output, adjustable wavelengths, priority zones, timers, and the like. The sensors of the sensor systems will enable the subject embodiments to have the ability to evaluate the skin of the face and body of a patient with sensors for color, wrinkles, age spots, acne, lesion density, and the like, and plan a smart treatment, utilizing more or less energy on the priority zones. The subject embodiments can be smart from the standpoint of skin type, age, overall severity of problems and have the ability to customize the treatment accordingly.

In yet another embodiment, the phototherapy system device includes an aligned eye slot disposed for user to see through the device. Also included is a radiation absorbing layer interposed between the lamps and the outer wall.

In yet another embodiment, the lamps are embedded in a flexible sheet of formable material and are integrally molded as strips within a material sheet.

In addition, control systems can measure or count device usage and communicate historical usage, and indicate a time for replacement.

The present disclosure thus describes a fully flexible and adjustable LED device which provides improved usability and light dispersion.

### BRIEF DESCRIPTION OF THE DRAWINGS

FIGURE 1 is a perspective view of one embodiment of a therapeutic lamp platform comprising a wearable mask;
FIGURE 2 is another perspective view of the device of Fig. 1;
FIGURE 3 is an exploded perspective view of Fig. 1;
FIGURE 4 is an exploded perspective view of Fig. 2;
FIGURE 5 is an exploded perspective view of the controller B;
FIGURE 6 is a cross-sectional view showing a two-wall structure of the embodiment of Fig. 1 wherein an inner wall includes light apertures aligned with the LEDs for communicating the therapeutic light to the user;
FIGURE 7 is a second cross-sectional view taken along a vertical center-line;
FIGURE 8 is a partial cross-sectional perspective view illustrating disposition of recessed LED lamps relative to inner wall apertures;
FIGURE 9 is a perspective view of an alternative embodiment wherein the power supply and control circuitry are integrally formed with the mask assembly;
FIGURE 10 is an exploded view of the device of Fig. 9;
FIGURE 11 is an exploded view of an alternative embodiment wherein the mask walls are spaced by a flange;
FIGURE 12 is an embodiment of a packaging assembly containing the device of Fig. 1;

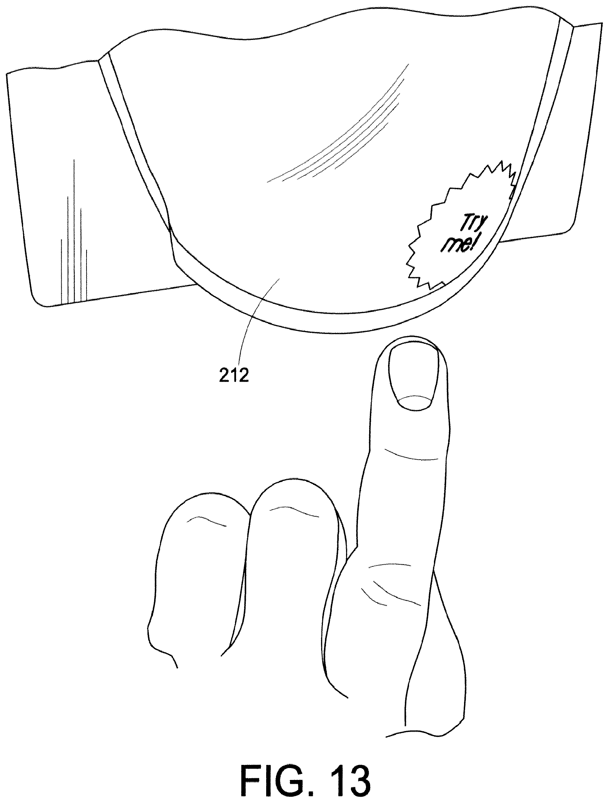

FIGURE 13 illustrates a try-me feature of the packaging of Fig. 11 wherein a user can view a sample operation of the device;
FIGURE 14 is a flowchart of operational device control;
FIGURE 15 is an exploded view of an alternative embodiment including a see-through slot and a third light absorbing layer;
FIGURE 16 (A) (B) (C) (D) and (E) are elevated views of the assembled device of FIGURE 15;
FIGURE 17 is an exploded view of an alternative embodiment including eye protecting goggles; and
FIGURE 18 is an exploded view of an alternative embodiment having a mask sized for applying the LED therapy to the eye area.

### DETAILED DESCRIPTION

The subject embodiments disclosed herein relate to a phototherapy system including methods and devices, preferably comprising a wearable hands-free device with a remote battery pack for powering therapeutic lamps in the device. The subject devices display numerous benefits including a light platform wherein the platform and the lamps therein are properly positionable relative to a user during use with no human touch. That is, structural componentry of the device not only supports the lamp platform on the user, but functions as a guide for the appropriate disposition of the lamps relative to the treatment areas of the user. The structural assembly of the device precludes sharp or hot surfaces from being engageable by a user as the lamps are recessed relative to an inner reflective surface closest to and facing the patient treatment surface. Circuit componentry to communicate power to the lamps is also encased within the wall structure. Therapeutic light, shining through wall apertures, is communicated to the user while the lamps and the circuitry are effectively encased within the spaced wall structure. A smooth seamless surface is thus presented to the user that is properly spaced for the desired therapeutic treatments, yet provides improved ventilation so that an aesthetic and appealing device surface is presented to the user that minimizes user discomfort. Other benefits relate to the adjustability of the device in the form of a flexible mask which forms upon user receipt to match a treatment surface, e.g., a head size, of the user. Smart componentry not only measures device usage, but may also calculate lamp degradations so that a time for proper replacement can be communicated to a user. The overall assembly is purposefully constructed of relatively light weight and minimized componentry for ease of user use and comfort.

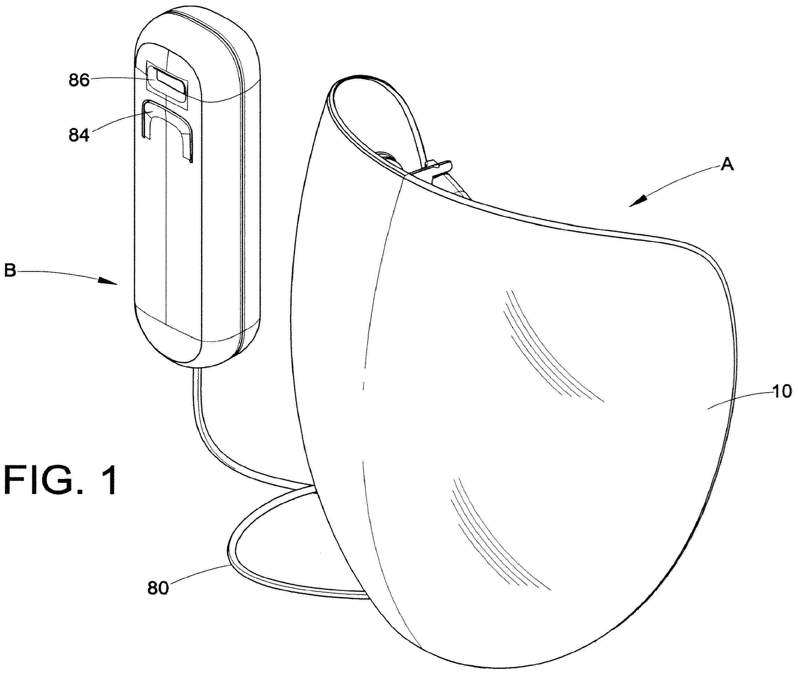

More particularly, and with reference to Figs. 1-4, subject embodiments preferably comprise a lamp platform **A** and a remote battery pack **B.** The platform **A** is comprised of a wall structure **10** encasing the plurality of therapeutic lamps such as red and blue LEDs **12** and circuitry **14** for communicating power to the lamps via cable **80** and connector **83** from the battery pack B. Other radiant energy forms could also include fluorescents, lasers or infrareds. The wall structure **10** is mounted on a support frame **20** connected via snap-out pivotal connections **22** which allows the wall structure to adjust position via a slight pivot relative to the frame **20.** The frame **20** also includes protective lenses **24** and a nose bridge **26.** The temple arms **28** may be fixed or telescopic and hinge relative to the frame **20** so that the platform A can be mounted on a user in a hands-free support manner via resting on the nose with the nose bridge **26** and the ears with temple arms **28.**

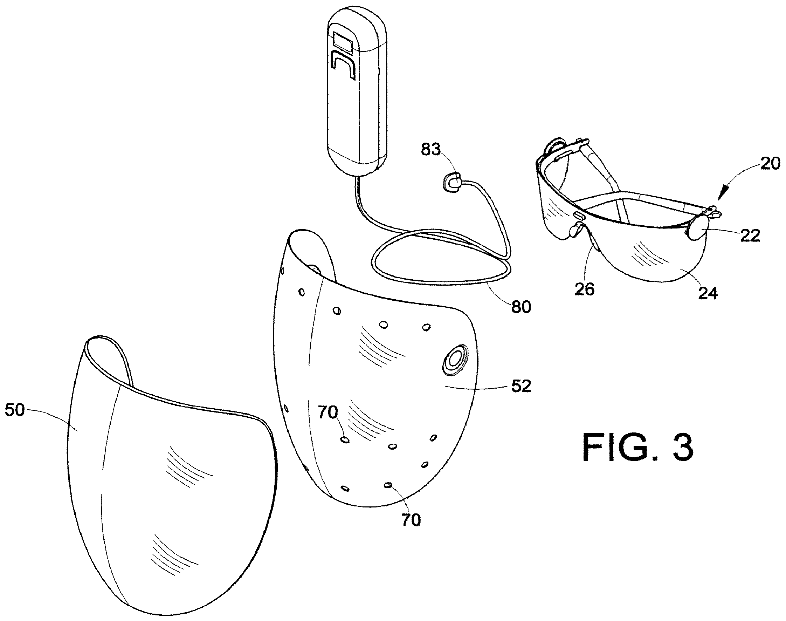

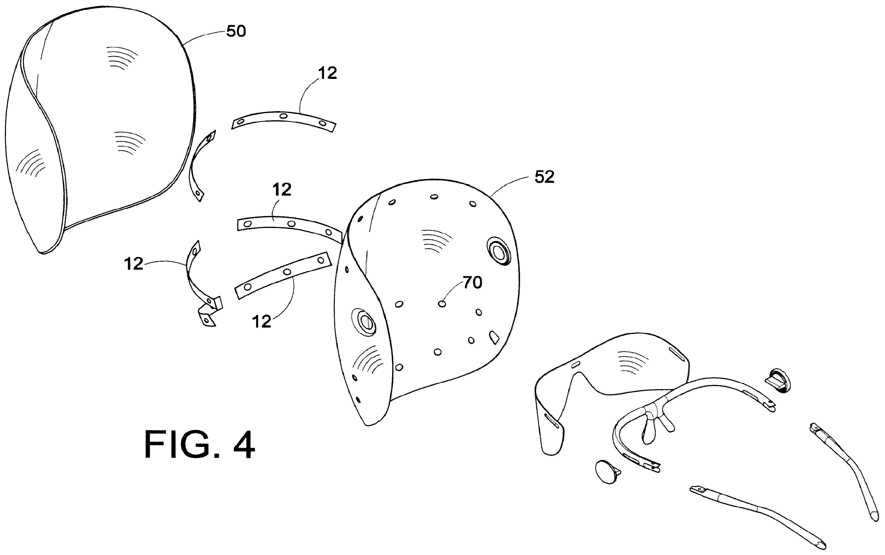

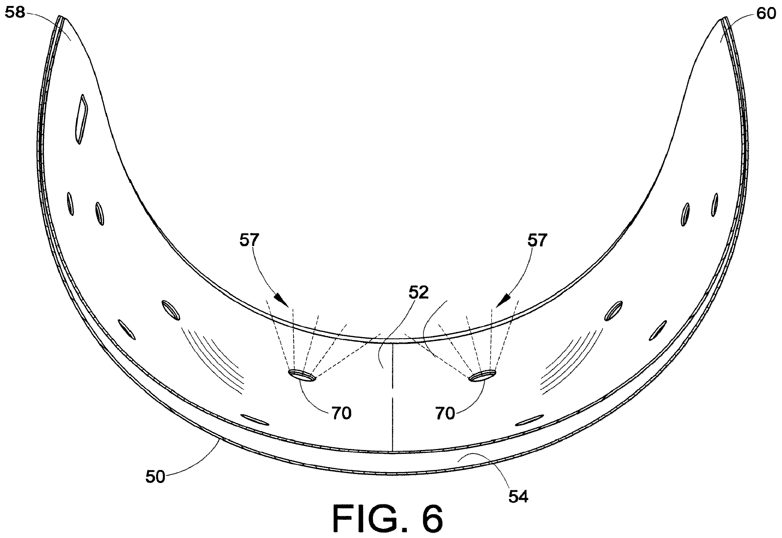

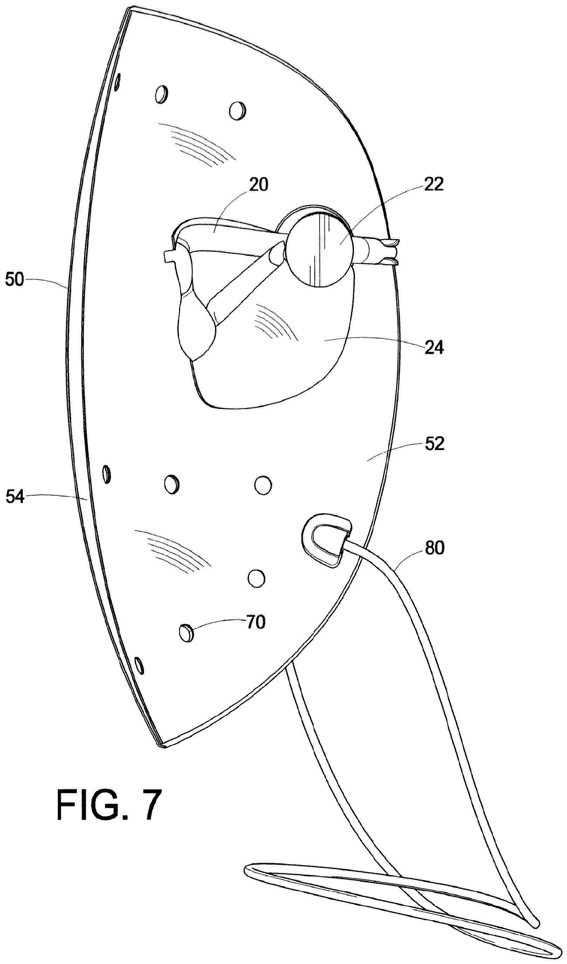

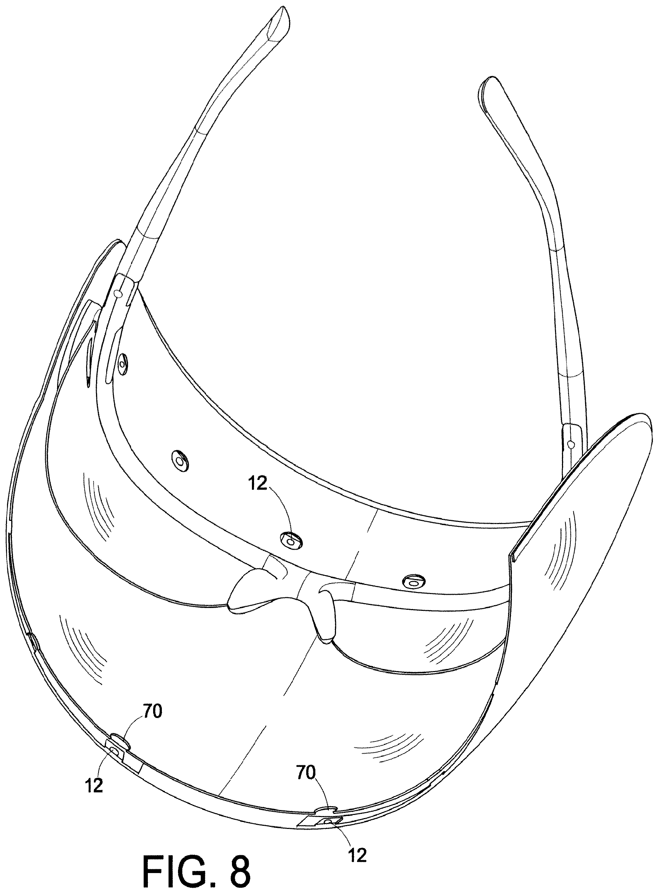

With reference to Figs. 3, 4, 6, 7 and 8 it can be seen that the wall structure **10** is comprised of an outer wall **50** and an inner wall **52.** The outer wall is disposed furthest away from the treatment surface of the user, while the inner wall **52** is disposed closer thereto. The walls have a concave configuration in both horizontal and vertical directions and are constructed of a plastic material having a malleable rigidity so that the structure **10** can be bent and deflected slightly during use. The concavity comprises a multi-dimensional parabolic curvature for catching and reflecting the radiation back to the treatment areas. It is intended that the concavity is slightly smaller than the head of the user so that the mask has to be bent out when applied thereby providing a close but comfortable tightness on the user which will keep the assembly **A** in a desired position during use. The concavity also positions the therapeutic lamps or LEDs **12** in desired positions relative to the user. The spacing **54** between walls **50** and **52** receives the lamps **12** and circuitry **14** so that the lamps and circuitry are interposed between the walls for enhanced safety and convenience purposes. It can be seen that the spacing is diminished from the middle of the device towards the end portions **58, 60;** however, the entire end perimeter of the assembly **10** is sealed as the walls come together. Such a mating seal is typically effected through a sonic weld arrangement. Alternatively, local sealing points (not shown) can be employed to assemble the walls together with spaced intermediate seals. Thus, the inner and outer masks have different radii of concavity but present an integral structure as far as the user is concerned. The outer wall **50** primarily functions as a support for the lamps **12** and circuitry **14.** With reference to Fig. 4 it can be seen that the lamps are disposed on the wall **50** in a predetermined manner for radiating treatment areas most susceptible for the phototherapeutic treatment. A minimum number of lamps **12** are intended but still enough to provide effective therapy. Alternatively, the lamps could be fixed to the inner wall **52.** Regardless of which wall supports the lamps, the lamps need to be properly aligned with apertures **70** to desired treatment areas.

Rather than placing a plurality of LEDs randomly, the subject LEDs are specifically minimized in number and disposed relative to the treatment areas and wall parabolic reflectivity to effect the desired therapy. More particularly, it can be seen that the individual lamps **12,** and associated inner wall apertures **70,** are disposed to treat the most common areas benefiting from the therapy. The present embodiments illustrate a placement pattern useful for skin acne treatment. Other placement patterns are certainly intended to fall within the scope of the disclosed embodiments. Here three LED strips are seen and would typically comprise two blue strips on the top and bottom of a middle red strip, as these frequencies are most useful for acne treatment. The subject invention may include only blue, only red, or any other mixed combination of LED or other radiant energy form pattern. The illustrated pattern would thus have intensified therapeutic effect on the jaw line, chin, cheek and forehead, but not the eyelids. Light sources can include LEDs, fluorescents, lasers or infrareds as an example. Such sources can vary in the form of the radiant energy delivery. Pulsed light (IPL), focused light (lasers) and other methods of manipulating light energy are encompassed within the present embodiments. Other methods of light emission may comprise continuous, pulsed, focused, diffuse, multi wavelength, single wavelength, visible and/or non-visible light wavelengths.

The inner wall **52** is comprised of a smooth seamless reflective surface facing the treatment area and includes a plurality of apertures **70** matingly aligned relative to the lamps so that the lamps can radiate the therapeutic light through the apertures **70.** Accordingly, the LEDs **12** are recessed relative to the inner wall **52** to preclude contact with the treatment surface and to make it very difficult for the lamps themselves to be in any way contacted by the user. Such an assembly results in a controlled communication of radiating therapy in a manner to impart a predetermined cone of therapeutic light on to a treatment area. The apertures are disposed relative to desired treatment areas and wall parabolic configuration for even light distributions across the treatment area. A combination of such a controlled cone of light, predetermined disposition of the lamps themselves on the platform, an inner reflective surface on the inner wall **52,** and a controlled positioning of the assembly relative to the treatment area via a platform position relative to contact areas of the nose and the ears, presents an assembly which presents a highly predictable distributive pattern of the light (predetermined cones of light per light source), thereby minimizing the number of lamps 12 that need to be included for effective treatment.

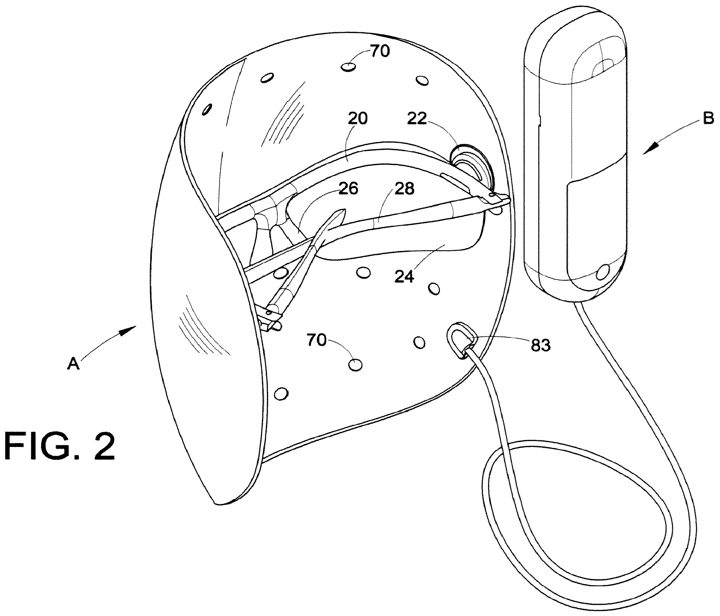

With reference to Figs. 2, 3 and 4, one embodiment comprises a support frame essentially comprising eyeglass frames as the associated support structure for the platform **10.** Interchangeable lenses **24** can be used to adjust the level of protection afforded by the lenses or their relative shape. Although not shown therein, telescopic temple arms **28** may telescope for better sizing relative to the head size of the user. Formable ear latches can also be included as part of the temple arms. Alternatively, the arms could include a head strap. The pivotable joints **22** allow the wall structure to pivot relative to the frames so that a user may adjust light intensity relative to a treatment area by moving the layers closer or farther away. As noted above, the platform **10** is flexible with a concave parabolic bias, but still has a malleable rigidity. When the frame **10** is received on the user, it is disposed to expand the platform parabolic bias to form a match to the size of the user. Eyeglass frame reference contact points of the user may comprise the nasion area, the nose bridge and the ears of the user. Alternatively, the support frame can comprise a goggle and head strap configuration relying on the nasion area.

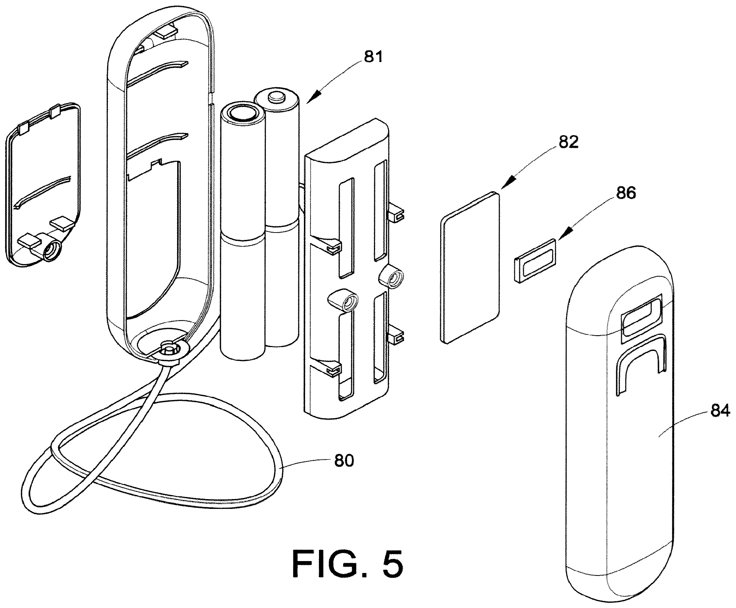

Battery pack **B** (Fig. 5) holds the supply batteries **81** and processing controller **82** that is in electrical communication with the lamps through wire **80.** The wiring between connectors **83** and LED strips **12** is not shown to avoid drawing clutter but is contained between walls **50, 52.** The battery pack will include an on-off switch **84** and a user interface **86.** The processing controller **82** may include a variety of control systems indicating device usage to the user. Such a system would be a counter. The user interface may comprise a display for a variety of useful information from the controller control systems to the user, such as a count of the number of times of usage and communication that the device has been used enough times such that the LEDs themselves have degraded and a replacement is recommended for the therapy.

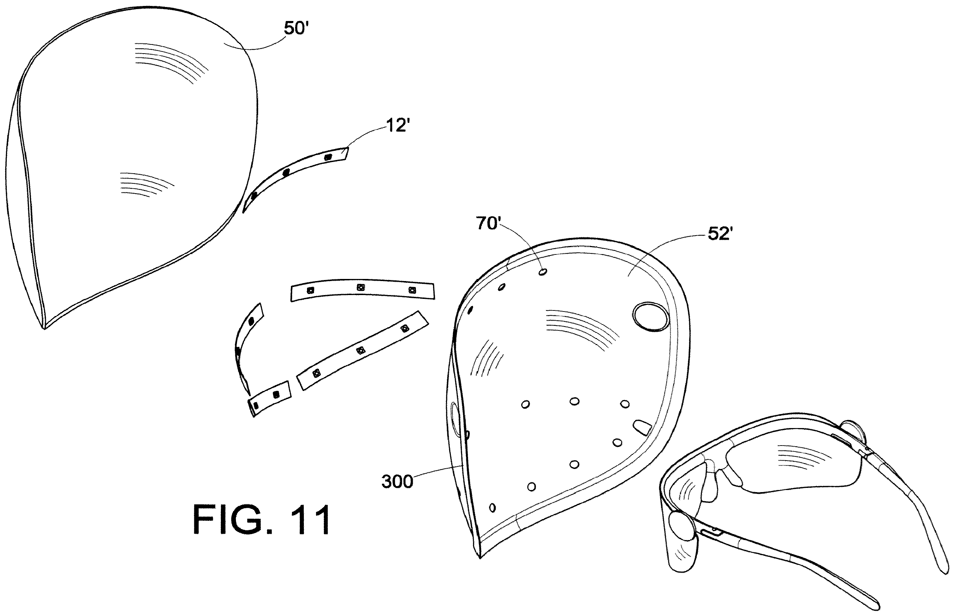

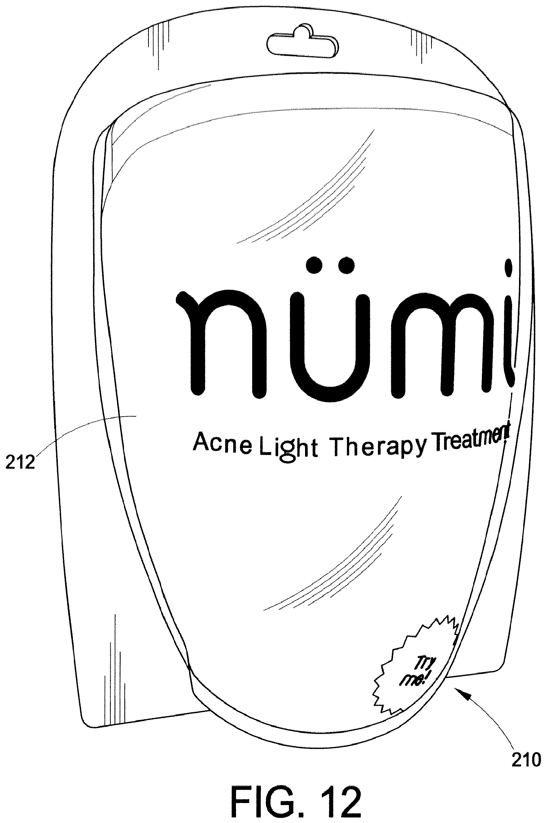

"Try-me packaging", Figs. 11 and 12, presents a demonstrative use opportunity to a potential user while still packaged. The subject embodiments further include a packaging assembly **210** containing the device wherein a switch **S1** (not shown) for operating the lamp assembly has a multi-position effect functionality including an on-mode, an off-mode and a try-me mode. The try-me mode is accessible while the lamp assembly is contained in packaging for displaying lamp operation to a user. The packaging includes a clear or translucent cover **212** over the device **A.** A try-me time-out circuit is included for limiting the try-me display time of lamp operation, such as, for example two seconds. Lamp on-time as measured by the counter is segregable from the try-me mode so that try-me usage will not affect dosage count of the device for actual therapy. It is assumed try-me usage time will be negligible relative to a dosage use time.

The subject devices include multiple benefits to the user in a wearable hands-free device with a remote battery pack. The device is properly positionable in a relatively automatic way with minimal human touch by exploiting user reference contact points, and is particularly hand-free during use. No sharp or hot surfaces are engageable by the user. A smooth seamless surface faces the user and is properly spaced from the treatment area to provide enhanced ventilation and minimal discomfort during treatment.

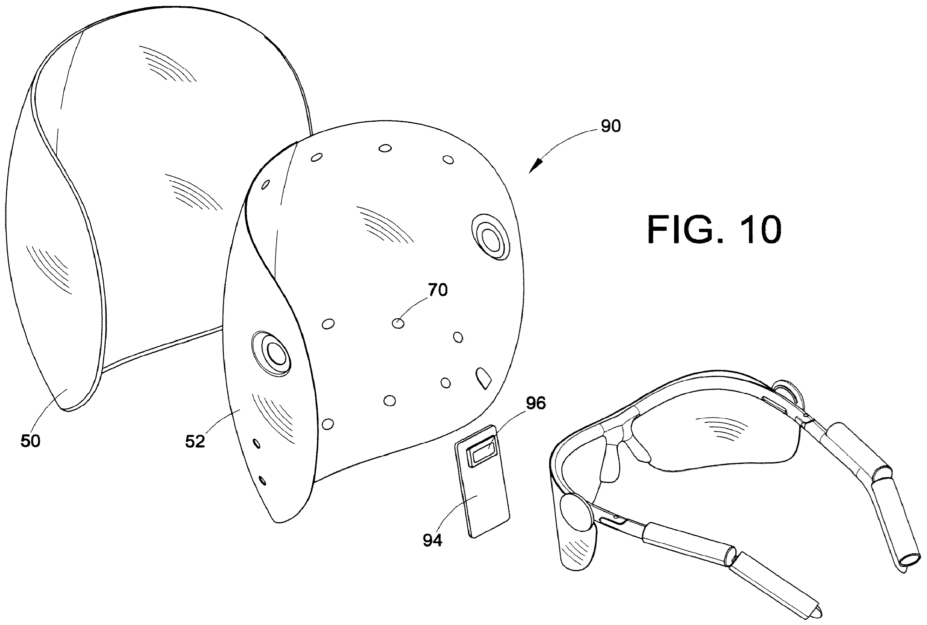

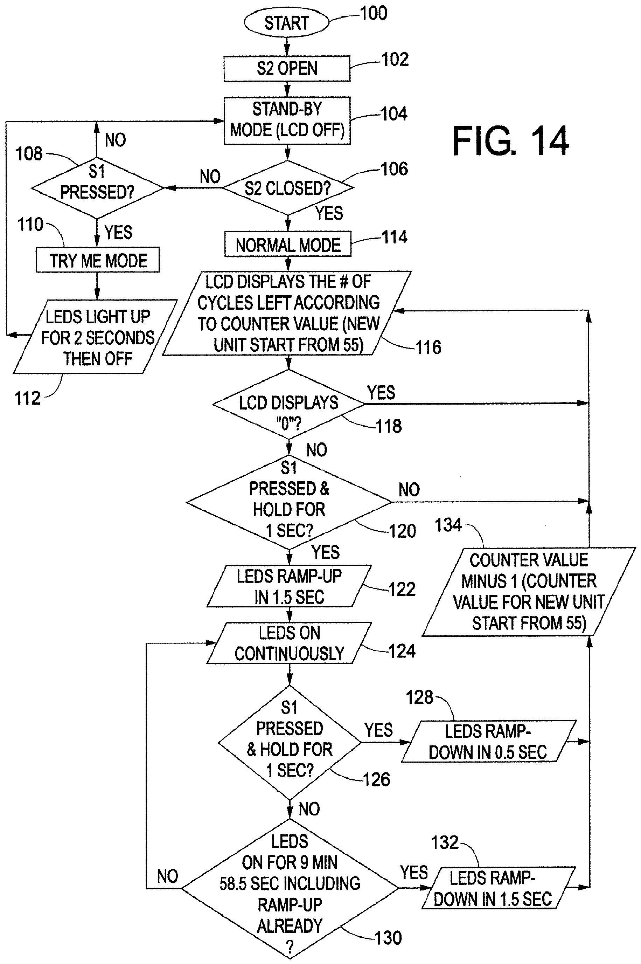

With particular reference to Fig. 14, a flowchart illustrating an operational embodiment of a device control is illustrated. The device visioned as operational by Fig. 10 includes two switches, **S1, S2,** at least one of which are required to be closed to communicate energy from an energy source to the therapeutic lamps. **S2** is a safety switch which is open when the device is in sales packaging so that only the "try-me" mode is enabled when **S2** is open. After removal from the packaging, **S2** can be closed and the device can be operated in a normal mode. Accordingly, after start **100,** and in a situation when **S2** is opened **102,** such as when the device is still within the packaging, the system will remain in a stand-by mode wherein the GUI interface (such as an LCD) is off **104.** If **S2** remains closed **106** but **S1** is pressed **108** (e.g. Fig. 12), then the device can enter the "try-me" mode **110** wherein the LEDs will light up for two seconds, then turn off **112.** Such a "try-me" mode operational demonstration to a user while the device is in a packaging communicates to the user actual operation and can assist in a decision to purchase, or have a better understanding of how the device operates. If the device is removed from the packaging, and **S2** is closed, the device will enter normal mode **114** wherein the GUI will include an LCD displaying the number of cycles left according to a counter value. Note that counter value **134** is not affected by any try-me sampling operation.

In one embodiment, the unit will count down from 55 to 1, as 55 uses is deemed to be enough to diminish enough LED efficiency from the peak operational mode of LEDs when they are used as the therapeutic radiant lamps. Accordingly, upon a user picking up the device, they will immediately know how many cycles are left for acceptable and recommended operation of the device from 55 more uses all the way down to 0 **118.** If the display shows a count greater than 0, and the user is interested in a therapy session, the user will turn the unit on by pressing **S1 120** wherein the LEDs will ramp up to radiant operation **122** in approximately 1.5 seconds and then will radiate continuously **124** until either the user desires to turn off the unit by again pressing **S1 126** so that the LEDs can ramp down **128** or until a therapy session has timed out **130** such as for remaining radiant for approximately ten minutes. After completing an appropriate run time of a therapy session, the LEDs will ramp down **132** and the GUI display to the user will subtract 1 from the counter value **134.**

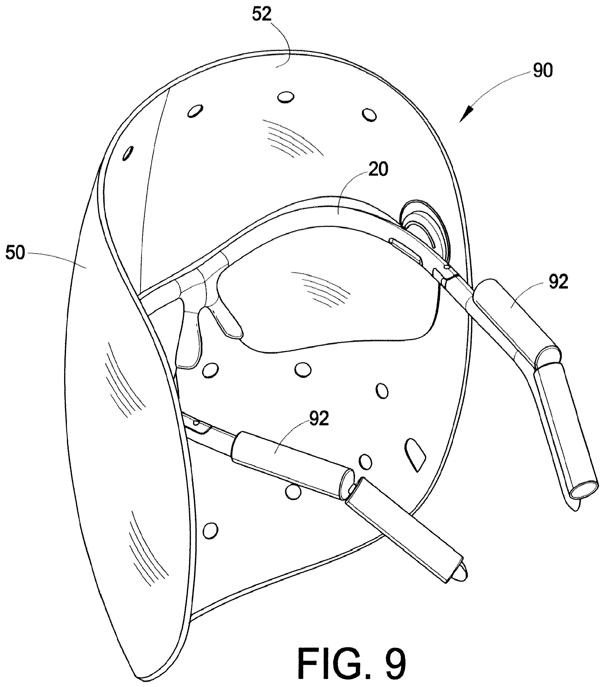

With reference to Figs. 9 and 10, an alternative embodiment is shown wherein a controller **B** is eliminated and the energy source and processing control are all integrally assembled in the device **90.** In this case, the platform **20** and walls **50, 52** remain substantially the same as per the Fig. 1 device. However, the energy source such as batteries **92** are disposed as part of the eyeglass temple arms wherein wires provide energy from the batteries **92** to the LEDs through the hinge points of the frame **20** and into the spacing **54** for ultimate connection to the LEDs themselves. The controller **94** including LCD display **96** is also housed behind the reflective wall **52** relative to the user, which wall **52** can include a relatively small cutout (not shown) for the screen **96.**

The embodiment of Figs. 9 and 10 is thus even more compact than the embodiment of Fig. 1, and more hands-free therefrom, as it eliminates the need to somehow manage the controller **B** during operation.

FIGURE 11 shows yet another alternative embodiment wherein the outer wall **50'** and the inner wall **52'** are not spaced by being configured with different curvatures. Rather, the walls **50', 52'** have the same curvature, but the inner wall **52** has an off step **300** depending from the wall perimeter to form a flange raised from the surface of the wall **52'** towards the outer wall **50'** to effectively form a spacer between the two. In one embodiment, the flange **300** is about 8 millimeters wide, continues around the entire perimeter of the wall **52'** and is about .5 millimeters thick for effecting the desired spacing between the inner and outer walls. In this embodiment the flange **300** is part of the inner wall **52',** and as in the foregoing embodiment, both walls are vacuumed formed plastic, either PET or PVC. The assembly of Fig. 11 can be sonic welded, glued, or adhered with double-sided adhesive. Alternatively, a plurality of intermediate sealing points (not shown) could be used instead of a continuous seal. In this embodiment it can be seen that there is an alternative number of LEDs **12'** opposite the forehead portion of the assembly relative to the user so that the number of apertures **70'** and LEDs **12'** are reduced from the foregoing embodiment from eighteen to fifteen. Either number are viable implementations of the desired therapy, although the other componentry of the assembly Fig. 11 is substantially the same as that shown in the foregoing Figs.

Another alternative embodiment from the device shown in Figs. 1, etc. includes disposition of a transparent flexible polymer sheet (not shown) incorporating working LED lights between outer wall **50** and inner wall **52.** Such a configuration would comprise the polymer film being coated with a transparent thin layer of carbon nanotubes in a specific configuration to act as the wire pathways to connect LED lights. The polymer would protect the LEDs from user contact. Such protective polymers are available under the Lumisys brand.

Yet another alternative embodiment includes such a transparent flexible polymer sheet wherein a reflective film is applied on top of the flexible polymer sheet including cutouts opposite the LEDs for allowing the radiant light to communicate through a reflective area in a manner as shown in the relationship of Fig. 4 between the LEDs' **12** inner wall **52** through aperture **70.** This arrangement may also include a flexible outer wall **50** on the other side of the flexible polymer sheet to provide malleable rigidity to the film, reflective coating assembly.

Yet another alternative embodiment includes a plurality of sensors (not shown), such as temperature or radiant energy sensors, disposed relative to inner wall **52** to monitor radiant energy exposure of a user during therapy. If such exposure is deemed inappropriate for any reason, sensing thereof is recognized by controller **B** and the therapy can be halted.

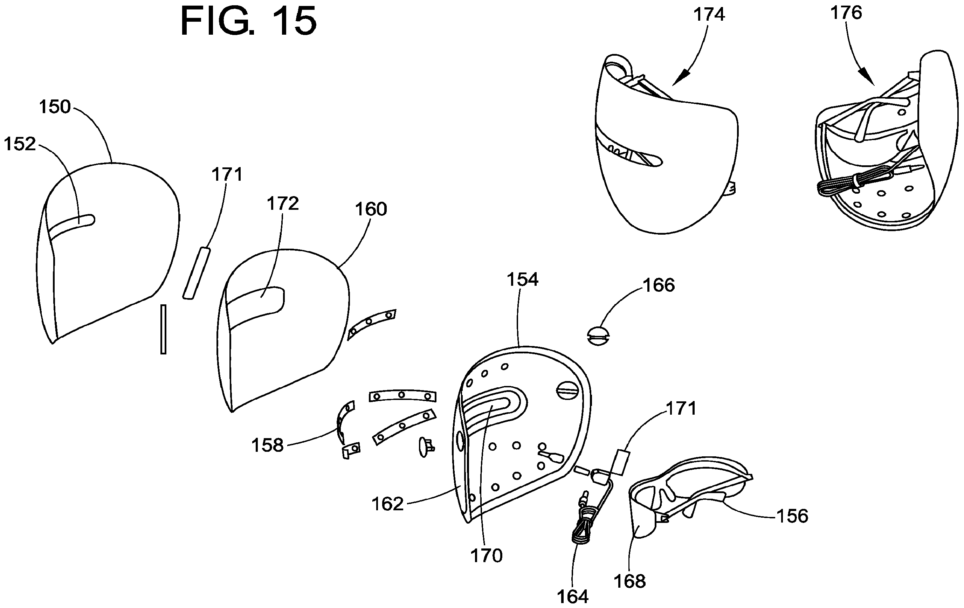

Fig. 15 shows yet another alternative embodiment including an outer shield **150** including a see-through slot **152,** an inner reflective shield **154,** and eyeglass assembly **156,** and LED strips **158.** These elements are substantially similar, but for the see-through slot **152** and corresponding aligned slots, as the foregoing embodiments. Alternatively, this embodiment includes a third layer **160** intermediate the outer shield **150** and the inner shield **154.** Layer **160** preferably comprises a thin opaque black plastic sheet which serves to absorb or block out lamp radiation and eliminate all light leakage from the front of the mask, i.e., out through the outer shield **150.** Layer **160** is preferably affixed to the inside of the outer layer **150** and then the LED strips are affixed to the layer **160.** The strips **158** still remain recessed relative to the inner surface **162** of the inner shield **154** for the benefits noted above. Fig. 15 also shows a controller assembly cable **164** and an eyeglass assembly mounting post **166.** The eyeglass assembly lenses **168** are tinted but do not preclude a user to see through the inner shield slot **170,** the third layer slot **172** and the outer shield see-through slot **152.** The aligned slots **152, 170, 172** comprise a continuous viewing opening that is an integral part of the mask. A layer **160** is sized to provide perimeter spacing from the outer perimeter of the outer shield **150.** When the unit is operating and the LEDs are illuminated, this provides a perimeter illumination to an observer of the user which not only communicates that the unit is in operation but provides an aesthetically pleasing appearance.

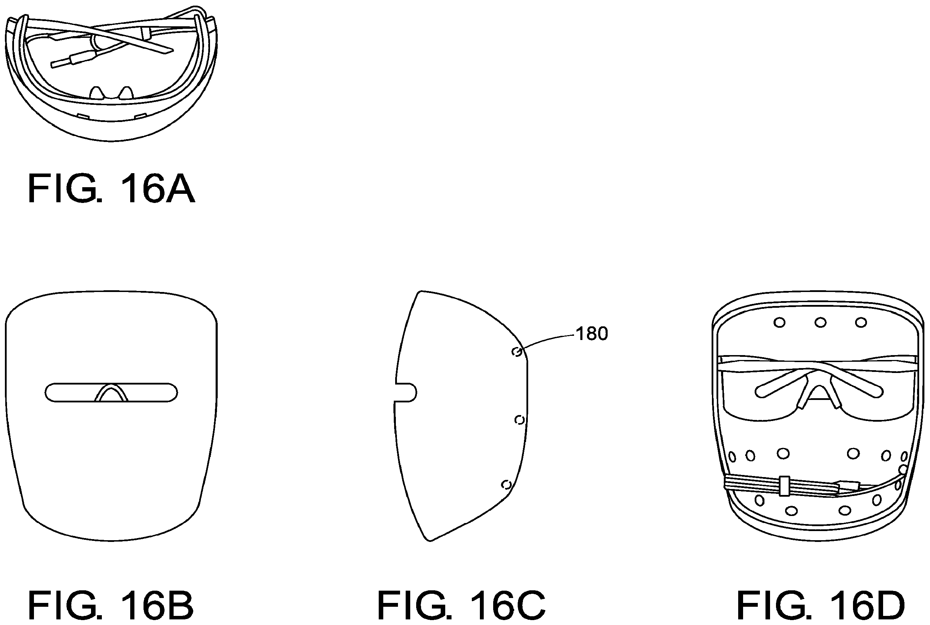

In one embodiment the LED strips **158** are preferably attached to the intermediate third layer **160** by being received in corresponding pockets (not shown) in the layer **160.** Alternatively, they can be adhesively applied to the layer **160.** The wires between the strips **158** are very thin and just rest between the middle layer and the inner shield **154,** i.e., no special wire routing. There is accommodation for the main cable and strain relief - leading to the first LED strip. The whole middle layer assembly fits into the chamfered recess in the inner shield **154,** and there are locating points top/bottom and left/right. This is secured with double-sided tape. The middle layer/LED strips/inner shield assembly is completed by the outer shield **150** (also by double-sided tape). There are several sonic welds **180** (Fig. 16) that permanently secure the layers together. Assembled perspective views **174, 176** are shown. Fig. 16 (A), (B), (C), and (D) illustrate elevated views of the embodiment of Fig. 15 when assembled.

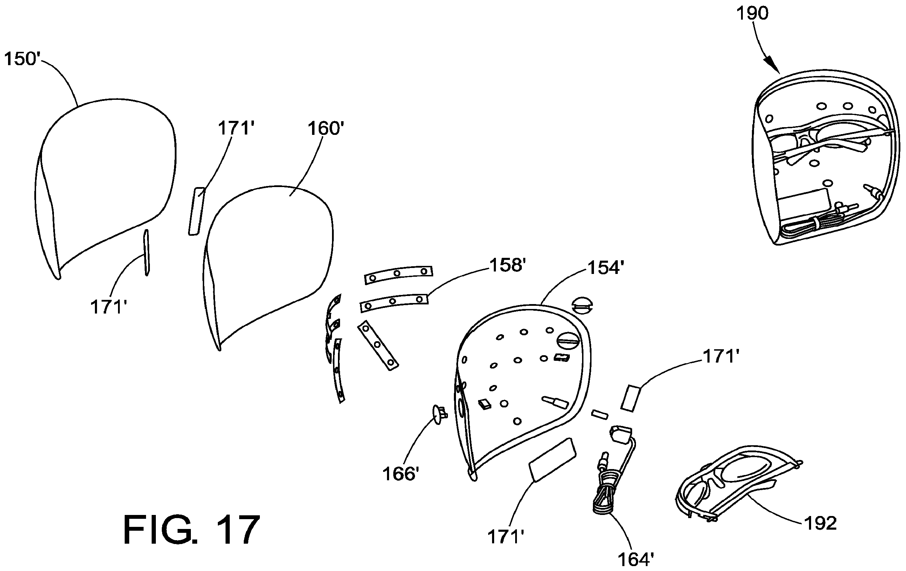

Fig. 17 is yet another alternative embodiment which differs from the embodiment of Fig. 15 in that the see-through slots **152, 170, 172** have been eliminated and the eyeglass assembly **190** no longer has tinted lenses, but radiant light blocking goggles **192.** Like elements from Fig. 15 are same numbered and primed. In this embodiment, the eyes are to be protected from any of the radiant energy emitted by the lamps. Such an embodiment is particularly useful for a phototherapeutic treatment of red and infrared light for an anti-aging therapy. A red light evens skin tone and reduces roughness. Infrared light reduces the appearance of fine lines and wrinkles. However, whatever radiant energy may be employed, the goggles **192** completely shield the eyes from the radiant energy.

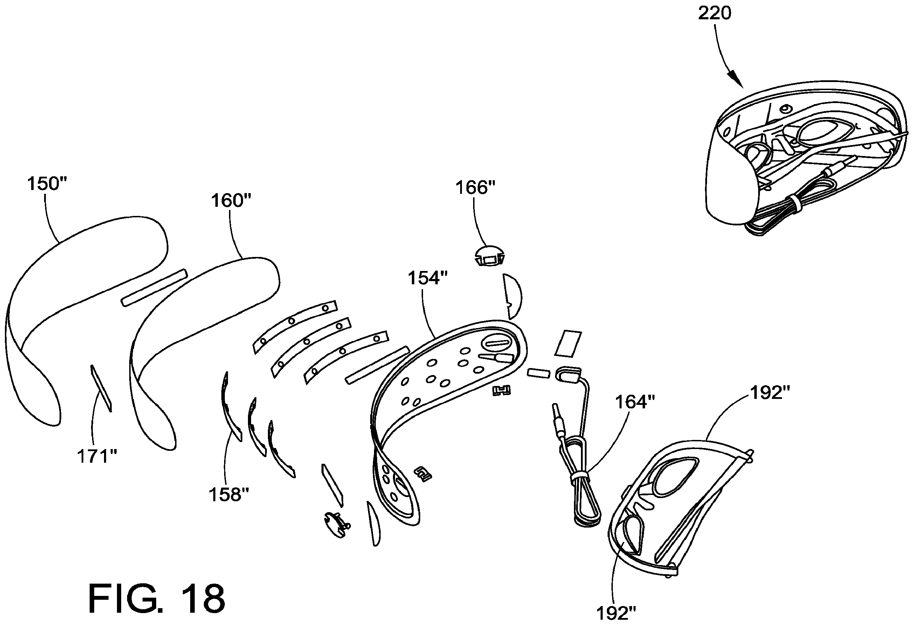

Fig. 18 is yet another embodiment where the mask assembly **220** is sized to only treat the eye area of a patient so that the assembled mask is much smaller than that shown in Fig. 17. The LED strips **158"** are disposed in a different arrangement from that Fig. 16 but the other elements are essentially the same including the protective goggles **192".**

It is a common feature of these embodiments though that the LED lamps remain recessed relative to the inner surface **162** of the inner shield **154** for comfort and safety purposes relative to the user.

It will be appreciated that variants of the above-disclosed and other features and functions, or alternatives thereof, may be combined into many other different systems or applications.

## Claims

1. A phototherapy system device comprising:
a plurality of therapeutic lamps (158);
a first wall (150);
a second wall (154) spaced from the first wall (150) and including a reflective surface and a plurality of light apertures substantially aligned with the lamps (158) for communicating lamp radiation to a user wherein the lamps are interposed between the first (150) and second walls (154); and
a radiation absorbing layer (160) interposed between the lamps (158) and the first wall (150) and wherein the radiation absorbing layer (160) has a peripheral edge spaced from a terminal peripheral edge of the first wall (150) for accommodating a visible radiation ring about the first wall terminal peripheral edge.

2. The device of claim 1 wherein the first (150) and second walls (154) include an aligned eye slot (152, 170, 172) disposed for a user to see through the device.

## Patentansprüche

1. Fototherapiesystemvorrichtung, die Folgendes umfasst:
mehrere therapeutische Lampen (158);
eine erste Wand (150);
eine zweite Wand (154), die von der ersten Wand (150) beabstandet ist und eine reflektierende Oberfläche und mehrere Lichtöffnungen, die im Wesentlichen mit den Lampen (158) ausgerichtet sind, zum Kommunizieren von Lampenstrahlung zu einem Benutzer beinhaltet, wobei die Lampen zwischen der ersten (150) und zweiten Wand (154) liegen; und
eine Strahlungsabsorptionsschicht (160), die zwischen den Lampen (158) und der ersten Wand (150) liegt, und wobei die Strahlungsabsorptionsschicht (160) einen Peripherierand, der von einem abschließenden Peripherierand der ersten Wand (150) beabstandet ist, zum Aufnehmen eines Sichtbare-Strahlung-Rings um den ersten abschließenden Wandperipherierand aufweist.

2. Vorrichtung nach Anspruch 1, wobei die erste (150) und zweite Wand (154) einen ausgerichteten Augenschlitz (152, 170, 172) beinhaltet, der so angeordnet ist, dass ein Benutzer durch die Vorrichtung hindurchsieht.

## Revendications

1. Dispositif de système de photothérapie comprenant :
une pluralité de lampes thérapeutiques (158) ;
une première paroi (150) ;
une deuxième paroi (154) espacée de la première paroi (150) et comportant une surface réfléchissante et une pluralité d'ouvertures lumineuses sensiblement alignées avec les lampes (158) pour la communication du rayonnement de la lampe à un utilisateur, les lampes étant interposées entre les première (150) et deuxième (154) parois ; et
une couche absorbant un rayonnement (160) interposée entre les lampes (158) et la première paroi (150) et la couche absorbant un rayonnement (160) possédant un bord périphérique espacé d'un bord périphérique terminal de la première paroi (150) pour l'accueil d'un anneau de rayonnement visible autour du bord périphérique terminal de la première paroi.

2. Dispositif selon la revendication 1, les première (150) et deuxième (154) parois comportant une fente oculaire alignée (152, 170, 172) disposée pour qu'un utilisateur voie à travers le dispositif.
